(19) Europäisches Patentamt
European Patent Office
Office européen des brevets

(11) Publication number: 0 053 017 B1

(12) EUROPEAN PATENT SPECIFICATION

(45) Date of publication of patent specification: 20.02.85

(21) Application number: 81305490.5

(22) Date of filing: 20.11.81

(51) Int. Cl.[4]: C 07 D 401/06, C 07 D 401/12, C 07 D 409/12, C 07 D 405/12, C 07 D 405/06, C 07 D 417/06, C 07 D 411/06, C 07 D 403/06, A 61 K 31/395

# (54) Amide derivatives.

(30) Priority: 24.11.80 GB 8037651

(43) Date of publication of application: 02.06.82 Bulletin 82/22

(45) Publication of the grant of the patent: 20.02.85 Bulletin 85/08

(84) Designated Contracting States: DE FR GB

(56) References cited:
EP-A-0 010 347
EP-A-0 021 883
EP-A-0 045 161
FR-A-2 340 933
GB-A-2 028 794
US-A-3 526 663

(73) Proprietor: IMPERIAL CHEMICAL INDUSTRIES PLC
Imperial Chemical House Millbank
London SW1P 3JF (GB)

(72) Inventor: Davies, David Huw
11, Farfields Close Gawsworth
Macclesfield Cheshire (GB)
Inventor: Preston, John
5, Ashworth Park
Knutsford Cheshire (GB)
Inventor: Walker, Edward Raymond Halstead
4, Capesthorne Road
Wilmslow Cheshire (GB)
Inventor: Ratcliffe, Arnold Harry
63 Dickens Lane Poynton
Stockport Cheshire (GB)

(74) Representative: Slatcher, Reginald Peter et al
Imperial Chemical Industries PLC Legal
Department: Patents PO Box 6
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 053 017 B1

Courier Press, Leamington Spa, England.

**Description**

This invention relates to new amide derivatives and more particularly it relates to amide derivatives which are inhibitors of angiotensin converting enzyme (hereinafter ACE).

Various inhibitors of ACE are known. One is commercially available under the name captopril and has the chemical structure:—

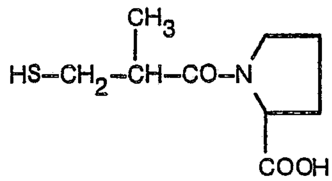

Another, known as MK 421, is at an advanced stage of clinical trial and this has the chemical structure:—

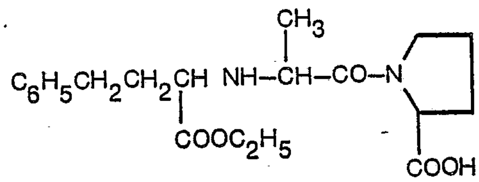

Various analogues of these compounds are described in the literature, especially in patent specifications, for example in French Specification No. 2,340,933; European Specification No. 10347 describes captopril analogues bearing both a thiol group and a ketonic group.

According to the invention there is provided an amide derivative of the formula:—

$$R^1—Y—NR^2—CHR^3—X—CHR^{11}—CHR^4—CO—NR^5—CR^6R^{16}—Q—R^{10}$$

wherein either $R^1$ is hydrogen, or alkyl of up to 15 carbon atoms which is unsubstituted, or which bears one substituent selected from hydroxy, alkoxy, alkylthio and alkoxycarbonyl each of up to 5 carbon atoms, and aryl, aryloxy, arylthio and heterocyclic substituents; or which bears two or three substituents one of which is aryl, another of which is hydroxy, amino, trifluoromethyl, aryloxy or alkoxy of up to 5 carbon atoms and the third of which, if present, is aryl or trifluoromethyl;

or $R^1$ is aryl or, when Y is carbonyl or thiocarbonyl, is aryloxy, alkoxy or arylalkoxy wherein the alkoxy part has up to 5 carbon atoms;

or $R^1$ is halogenoalkyl of up to 6 carbon atoms or is alkenyl, or cycloalkyl each of up to 6 carbon atoms which is unsubstituted or which bears an aryl substituent;

or $R^1$ is a substituent of the formula $R^8CONHCHR^9$— or $R^8COCH_2CHR^9$— wherein $R^8$ is alkyl or cycloalkyl each of up to 10 carbon atoms, or aryl and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally-occurring amino acid;

or $R^1$ is heterocyclic;

wherein $R^2$ is hydrogen, alkyl of up to 5 carbon atoms which is unsubstituted or which bears an aryl substituent, or aryl;

wherein $R^3$ is alkyl or alkenyl each of up to 5 carbon atoms which is unsubstituted or which bears an aryl substituent;

or $R^3$ is aryl or indolylmethyl or $R^3$ is alkyl of up to 5 carbon atoms which is substituted by heterocyclic, other than methyl substituted by indolyl;

provided that when $R^1$ is other than heterocyclic or alkyl substituted by heterocyclic, $R^3$ must be alkyl substituted by heterocyclic other than methyl substituted by indolyl;

wherein $R^4$ is hydrogen or alkyl of up to 5 carbon atoms which is unsubstituted or which bears an aryl substituent;

wherein either $R^5$ is hydrogen or aryl, or alkyl of up to 5 carbon atoms which is unsubstituted or which bears an aryl substituent;

or $R^5$ is joined together with $R^6$ as defined below;

wherein either $R^6$ is hydrogen, aryl or heterocyclic, or alkyl of up to 5 carbon atoms which is unsubstituted or which bears a hydroxy, aryl or heterocyclic substituent;

or $R^6$ and $R^5$ are joined together to form alkylene or alkenylene of 2 to 5 carbon atoms; or an oxa-, thia- or aza-derivative of said alkylene or alkenylene; or an oxo-substituted derivative of said alkylene or alkenylene;

or $R^6$ and $R^{16}$, or $R^6$, $R^{16}$ and $R^5$, or $R^6$ and $R^{10}$ are joined together as defined below;

wherein $R^{16}$ is hydrogen or alkyl of up to 5 carbon atoms;

or $R^6$ and $R^{16}$ are joined together to form alkylene of 2 to 5 carbon atoms (that is, to form a spirocycloalkyl group);

or $R^{16}$ together with the first carbon atom of $R^6$ form a double bond wherein $R^6$ is otherwise alkyl, or wherein $R^6$ is otherwise substituted alkyl as defined above, or wherein $R^6$ and $R^5$ are otherwise joined together as defined above (that is, so that $R^5$, $R^6$ and $R^{16}$ together form alkylidene);

wherein Q is carbonyl (—CO—) or methylene (—$CH_2$—);

and wherein either $R^{10}$ is hydroxy, amino, aryloxy, arylthio, alkoxy, cycloalkoxy, alkylamino, dialkylamino, cyclic amino, hydroxyalkoxy, acyloxyalkoxy, aminoalkoxy, alkylaminoalkoxy, dialkylaminoalkoxy, cyclic aminoalkoxy or arylalkoxy wherein each alkyl or alkoxy has up to 5 carbon atoms and wherein cyclic amino has up to 6 carbon atoms;

or wherein $R^{10}$ and $R^6$ are joined together such that $R^{10}$ is oxygen (—O—) joined to the terminal carbon atom of $R^6$ when it is alkyl;

wherein $R^{11}$ is hydrogen or alkyl of up to 3 carbon atoms;

wherein X is carbonyl (—CO—), hydroxymethylene (—CHOH—), thiocarbonyl (—CS—) or oximinomethylene (—C=N—$OR^{20}$, wherein $R^{20}$ is hydrogen or alkyl of up to 5 carbon atoms);

and wherein Y is carbonyl, thiocarbonyl, sulphonyl (—$SO_2$—) or amido (—NHCO—);

or a salt thereof where appropriate.

It will be observed that there are various potentially asymmetrical carbon atoms in the amide of the invention, in particular the carbon atoms which bear the substituents $R^3$, $R^4$ and $R^{11}$ when these substituents are other than hydrogen, and the carbon atom which bears the substituents $R^6$ and $R^{16}$ when these are different one from the other, and that the amide may therefore exist in racemic and optically active forms. It is to be understood that this invention encompasses the racemic form and any optically-active form which possesses ACE-inhibiting properties, it being a matter of common general knowledge how an optically active compound may be prepared and how the ACE-inhibiting properties of a compound may be measured.

A suitable value for $R^1$ or $R^8$ when it is alkyl is, for example, methyl, isobutyl or n-tridecyl.

A suitable value for $R^2$, $R^3$, $R^4$, $R^5$, $R^6$, $R^9$, $R^{16}$ or $R^{20}$ when it is alkyl is, for example, methyl, ethyl, n-propyl or isobutyl.

A suitable value for $R^{11}$ when it is alkyl is, for example, methyl or ethyl.

A suitable value for $R^1$ or $R^{10}$ when it is alkoxy, or for the alkoxy substituent in $R^1$ when said group is alkyl substituted by alkoxy is, for example, methoxy, ethoxy or t-butoxy.

A suitable value for $R^1$ or $R^3$ when it is alkenyl, is, for example, allyl or 2-methylprop-2-enyl.

A suitable value for $R^1$ or $R^8$ when it is cycloalkyl is, for example, cyclopropyl, cyclopentyl or cyclohexyl.

A suitable value for $R^1$, $R^2$, $R^3$, $R^5$, $R^6$ or $R^8$ when it is aryl, or for the aryl substituent in the group $R^1$, $R^2$, $R^3$, $R^4$, $R^5$, $R^6$ or $R^9$ when said group is alkyl substituted by aryl, or for the aryl substituent in $R^1$ when said group is alkenyl, halogenoalkenyl or cycloalkyl substituted by aryl, or for the aryl substituent in $R^3$ when said group is alkenyl substituted by aryl, or for the aryl substituent in $R^1$ or $R^{10}$ when it is aryl-alkoxy, that is, alkoxy substituted by aryl, is, for example, unsubstituted phenyl or naphthyl, or phenyl or naphthyl substituted by one or more substituents selected from halogen, for example, fluorine, chlorine, bromine and iodine, alkyl, alkenyl, alkynyl, alkylthio and alkoxy each of up to 5 carbon atoms, for example methyl, ethyl, t-butyl, allyl, propargyl, methylthio, methoxy and ethoxy, and hydroxy, methylenedioxy, amino, nitro, cyano, carboxy, carbamoyl, trifluoromethoxy and trifluoromethyl, aryl, for example phenyl and *p*-chlorophenyl, and substituents of the formula

| | | |
|---|---|---|
| —$COR^{30}$ | —$NHR^{30}$ | —$NR^{30}R^{31}$ |
| —$COOR^{30}$ | —$CONHR^{30}$ | —$CONR^{30}R^{31}$ |
| —$NHCOR^{30}$ | —$NHSO_2R^{30}$ | —$SO_2NR^{30}R^{31}$ |
| —$SR^{30}$ | —$SOR^{30}$ | —$SO_2R^{30}$ |
| —(alk)—$COR^{30}$ | | |
| —(alk)—$COOR^{30}$ | | |

wherein either $R^{30}$ and $R^{31}$, which may be the same or different, each is phenyl, or trifluoromethyl, or alkyl of up to 5 carbon atoms, for example methyl, ethyl or n-propyl, which is unsubstituted or which is substituted by phenyl, or $R^{30}$ and $R^{31}$ are joined such that together with the adjacent nitrogen atom they form pyrrolidino, carboxypyrrolidino, alkoxycarbonylpyrrolidino, piperidino, 4-methylpiperazino or morpholino, and wherein —alk— is alkylene of 1 to 4 carbon atoms.

A suitable value for $R^1$ or $R^{10}$ when it is aryloxy, or for the aryloxy substituent in $R^1$ when $R^1$ is alkyl substituted by aryloxy, is, for example, unsubstituted phenoxy or phenoxy substituted by one or more substituents selected from those stated above as suitable substituents in aryl.

A suitable value for $R^{10}$ when it is arylthio, or for the arylthio substituent in $R^1$ when $R^1$ is alkyl substituted by arylthio is, for example, unsubstituted phenylthio or phenylthio substituted by one or more substituents selected from those stated in the penultimate paragraph as possible substituents in aryl.

A suitable value for the alkylthio or alkoxycarbonyl substituent in $R^1$ when it is alkyl substituted by alkylthio or alkoxycarbonyl is, for example, methylthio or ethoxycarbonyl.

A suitable value for $R^1$ when it is halogenoalkyl is, for example, trifluoromethyl.

A suitable value for $R^1$ or $R^6$ when it is heterocyclic, or for the heterocyclic substituent in $R^1$ or $R^6$ when it is alkyl substituted by heterocyclic, is, for example, a 5- or 6-membered heterocyclic ring containing 1 or 2 heteroatoms selected from oxygen, nitrogen and sulphur, which ring may be saturated or unsaturated, and which ring may optionally contain one or more oxo, alkyl or halogen substituents, for example methyl, chloro or bromo substituents, and which heterocyclic ring may also be fused to a benzene ring. Suitable heterocyclic rings are, for example, pyridyl, furyl, 5-methyl-2-furyl, thienyl, imidazolyl, thiazolyl, isoxazolyl, indolyl, benzofuryl, benzothienyl, quinolyl or benzimidazolyl.

A suitable value for the heterocyclic substituent in $R^3$ when $R^3$ is alkyl substituted by heterocyclic is, for example, any of the values stated in the last-mentioned paragraph, except that $R^3$ cannot be methyl substituted by indolyl.

A suitable value for alkylene or alkenylene formed by $R^5$ and $R^6$ joined together is, for example, ethylene, trimethylene, tetramethylene, pentamethylene, propenylene, ($-CH_2-CH=CH-$), but-1-enylene ($-CH_2CH_2-CH=CH-$), 2-oxatrimethylene ($-CH_2-O-CH_2-$), 2-thiatrimethylene ($-CH_2-S-CH_2-$), 3-oxatetramethylene ($-CH_2CH_2-O-CH_2-$), 3-thiatetramethylene ($-CH_2CH_2-S-CH_2-$), 3-azatetramethylene ($-CH_2CH_2-NH-CH_2-$), 3-methyl-3-azatetramethylene

$$(-CH_2CH_2-\overset{\displaystyle CH_3}{\overset{|}{N}}-CH_2-),$$

1-oxotrimethylene ($-COCH_2CH_2-$) or 1-oxotetramethylene ($-COCH_2CH_2CH_2-$).

A suitable value for alkylene formed by $R^6$ and $R^{16}$ joined together, is, for example ethylene, trimethylene or tetramethylene.

A suitable value for alkylidene formed by $R^5$, $R^6$ and $R^{16}$ joined together is, for example, propan-1-yl-3-ylidene ($-CH_2CH_2CH=$).

A suitable value for $R^9$ when it is a group derived from a naturally-occurring amino acid is, for example, 3-aminopropyl (from L-ornithine), 4-aminobutyl (from L-lysine), hydroxymethyl (from L-serine), α-hydroxyethyl (from L-threonine), β-hydroxyethyl (from L-homoserine), mercaptomethyl (from L-cysteine), β-methylthioethyl (from L-methionine), 3-guanidinopropyl (from L-arginine), imidazol-4-ylmethyl (from L-histidine), carboxymethyl (from L-aspartic acid) or 2-carboxyethyl (from glutamic acid).

A suitable value for $R^{10}$ when it is cycloalkoxy, hydroxyalkoxy or acyloxyalkoxy is, for example, cyclopentyloxy, cyclohexyloxy, 2-hydroxyethoxy, 3-hydroxypropoxy or pivaolyloxymethoxy.

A suitable value for $R^{10}$ when it is alkylamino, dialkylamino or cyclic amino is, for example, methylamino, ethylamino, dimethylamino, pyrrolidino, piperidino, 4-methylpiperazino or morpholino.

A suitable value for $R^{10}$ when it is aminoalkoxy, alkylaminoalkoxy, dialkylaminoalkoxy or cyclic aminoalkoxy, is, for example, ethoxy or propoxy substituted in the 2- or 3-position respectively by amino, methylamino, ethylamino, dimethylamino, pyrrolidino, piperidino, 4-methylpiperazino or morpholino. Alternatively, $R^{10}$ when cyclic aminoalkoxy may be a cyclic amine bearing an oxy-substituent on one of the carbon atoms of the cyclic amine, for example 1-methylpiperidin-4-yloxy.

When Q is carbonyl and $R^6$ and $R^{10}$ are joined together the group $-CR^6R^{16}-Q-R^{10}-$ may be, for example, the lactone group 2-oxotetrahydrofuran-3-yl or 2-oxo-tetrahydropyran-3-yl.

Preferably X, Y and Q are all carbonyl and $R^2$ and $R^{11}$ are both hydrogen.

One preferred amide derivative of the invention has the formula:—

$$R^1CONH-CHR^3-COCH_2-CHR^4-CONR^5-CHR^6-COR^{10}$$

wherein $R^1$ is methyl, benzyl, β-phenylethyl, benzyloxy, phenoxymethyl, phenylthiomethyl, phenyl, tolyl, methoxyphenyl, methylthiophenyl, monochlorophenyl, dichlorophenyl, fluorophenyl, iodophenyl, nitrophenyl, cyanophenyl, trifluoromethylphenyl, acetamidophenyl, acetylphenyl, methanesulphonylphenyl, biphenylyl, naphthyl, benzamidomethyl, cyclopentanecarbonamidomethyl or β-benzoylethyl;

$R^3$ is methyl substituted by a 5- or 6-membered, fully-unsaturated heterocyclic ring containing 1 or 2 hetero- atoms selected from oxygen, nitrogen and sulphur, which ring may optionally contain a methyl substituent or be fused to a benzene ring, provided that $R^3$ is not indolyl-methyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-thiatrimethylene, 3-thiatetramethylene, 3-

oxatetramethylene, 3-methyl-3-azatetramethylene; and $R^{10}$ is hydroxy, amino, alkoxy of up to 5 carbon atoms, especially methoxy, ethoxy, isopropoxy or t-butoxy, or β-dimethylaminoethoxy or β-morpholinoethoxy, or benzyloxy.

A particularly preferred amide derivative of this first group is one which has the last-mentioned formula wherein $R^1$ is phenyl, $R^3$ is methyl substituted by 3-thienyl, 5-methyl-2-furyl, 2-pyridyl, 3-pyridyl, 4-thiazolyl, 4-imidazolyl, 3-isoxazolyl or 2-benzimidazolyl, $R^4$ is hydrogen or methyl, $R^5$ and $R^6$ together form trimethylene and $R^{10}$ is hydroxy, alkoxy of up to 5 carbon atoms, especially methoxy, or benzyloxy.

A second preferred amide derivative of the invention has the formula

$$R^1CONH—CHR^3—COCH_2—CHR^4—CONR^5—CHR^6—COR^{10}$$

wherein $R^1$ is a 5- or 6-membered, fully-unsaturated heterocyclic ring containing 1 or 2 hetero-atoms selected from oxygen, nitrogen and sulphur, which ring may optionally contain a methyl substituent or be fused to a benzene ring;

$R^3$ is benzyl which is unsubstituted or bears a methyl, t-butyl, methoxy, fluoro, chloro, bromo, nitro, cyano, trifluoromethyl, amino, acetamido, methanesulphonamido, trifluoromethanesulphonamido or phenyl substituent, or

$R^3$ is 3-phenylpropyl or 3-phenylprop-2-enyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-thiatrimethylene, 3-thiatetramethylene, 3-oxatetramethylene or 3-methyl-3-azatetramethylene; and

$R^{10}$ is hydroxy, amino, alkoxy of up to 5 carbon atoms, especially methoxy, ethoxy, isopropoxy or t-butoxy, or β-dimethylaminoethoxy or β-morpholinoethoxy, or benzyloxy.

A particularly preferred amide derivative of this second group is one which has the last-mentioned formula wherein $R^1$ is 2-furyl, 2-thienyl or 2-pyridyl, $R^3$ is benzyl, $R^4$ is hydrogen or methyl, $R^5$ and $R^6$ together form trimethylene and $R^{10}$ is hydroxy or alkoxy of up to 5 carbon atoms, especially methyl or isopropyl.

Specific compounds of the invention are hereinafter described in the Examples, and of these preferred compounds are *N* - [2 - methyl - 4 - oxo - 6 - phenyl - 5 - (2 - thenoylamino)hexanoyl] - L - proline; *N* - [5 - (2 - furoylamino) - 4 - oxo - 6 - phenylhexanoyl] - L - proline isobutyl ester; *N* - [5 - benzamido - 4 - oxo - 6 - (pyrid - 3 - yl)hexanoyl] - L - proline and N - [5 - benzamido - 6 - (imidazol - 4 - yl) - 4 - oxohexanoyl] - L - proline.

A suitable salt of an amide derivative of the invention wherein $R^{10}$ is hydroxy is, for example, an alkali metal or alkaline earth metal salt, for example a sodium, potassium, calcium or magnesium salt, or an ammonium or dicyclohexylamine salt.

An amide derivative of the invention may be manufactured by any chemical process known to be suitable for preparing compounds of related chemical types.

A preferred process for the manufacture of an amide derivative of the invention comprises the reaction of an acid of the formula:—

$$R^1—Y—NR^2—CHR^3—X—CHR^{11}—CHR^4—COOH$$

wherein $R^1$, $R^2$, $R^3$, $R^4$, $R^{11}$, X and Y have the meanings stated above, or of a reactive derivative thereof, with an amine of the formula:—

$$HNR^5—CR^6R^{16}—Q—R^{12}$$

wherein $R^5$, $R^6$, $R^{16}$ and Q have the meanings stated above and wherein $R^{12}$ has any of the meanings stated above for $R^{10}$ except those containing a free hydroxy or amino group.

The abovementioned reaction may be carried out by reacting the acid and amine together in the presence of a carbodiimide, for example dicyclohexylcarbodiimide, and N-hydroxybenzotriazole, in a diluent or solvent, for example tetrahydrofuran, methylene chloride or dimethylformamide, at ambient temperature or below.

The acid starting material for the above reaction wherein $R^2$ is hydrogen and X and Y are both carbonyl, that is, having the formula:—

$$R^1CONH—CHR^3—CO—CHR^{11}—CHR^4—COOH$$

may be obtained by the reaction of a compound of the formula:—

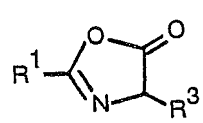

wherein $R^1$ and $R^3$ have the meanings stated above, with an acid chloride of the formula:—

$$ClCO—CHR^{11}—CHR^4—COOR^{13}$$

wherein $R^1$ and $R^{11}$ have the meanings stated above and wherein $R^{13}$ is alkyl of up to 5 carbon atoms, followed by the hydrolysis of the oxazolone ring and also hydrolysis to replace $R^{13}$ by hydrogen. The starting material wherein $R^2$ is alkyl, arylalkyl or aryl may be obtained by the reaction of the intermediate thus obtained, wherein $R^{13}$ is alkyl or has been replaced by hydrogen, with a compound of the formula $R^2Z$, wherein $R^2$ has the meaning stated above and wherein Z is a displaceable group such as a halogen atom or an alkanesulphonyl or arenesulphonyl group, for example a chlorine, bromine or iodine atom or a methanesulphonyl or toluene-*p*-sulphonyl group.

Alternatively, the starting material wherein $R^2$ is hydrogen and X is carbonyl or thiocarbonyl may be obtained by the reaction of a compound of the formula:—

$$H_2NCHR^3X^1CHR^{11}CHR^4COOR^{13}$$

wherein $R^3$, $R^4$, $R^{11}$ and $R^{13}$ have the meanings stated above and $X^1$ is carbonyl or thiocarbonyl, with a compound of the formula $R^1—Y—Z^1$, wherein $R^1$ and Y have the meanings stated above and wherein $Z^1$ is a displaceable group such as is defined above for Z, or $Z^1$ is hydroxy (in which case the reaction is carried out in the presence of a carbodiimide or an alkyl chloroformate), or, when Y is amido, with a compound of the formula $R^1NCO$, or, when Y is thiocarbonyl, with a compound of the formula $R^1CS—SCH_2COOH$.

An amide derivative of the invention wherein $R^2$ is alkyl, arylalkyl or aryl (and $R^{10}$ is $R^{12}$) may be obtained by reacting the corresponding amide derivative of the invention wherein $R^2$ is hydrogen (and $R^{10}$ is $R^{12}$) with a compound of the formula $R^2Z$, wherein $R^2$ and Z have the meanings stated above.

An amide derivative of the invention wherein Q is carbonyl and $R^{10}$ is hydroxy may be obtained by the hydrolysis of the corresponding amide derivative of the invention wherein $R^{10}$ is alkoxy, or, when $R^{10}$ is t-butoxy, by the acid-catalysed cleavage of said compound.

An amide derivative of the invention wherein X is hydroxymethylene may be obtained by the reduction, for example with an alkali metal borohydride, of the corresponding amide derivative of the invention wherein X is carbonyl, and an amide derivative of the invention wherein X is oximinomethylene may be obtained by the reaction of the corresponding amide derivative of the invention wherein X is carbonyl with hydroxylamine or an *O*-substituted hydroxylamine derivative.

As stated above, an amide derivative of the invention possesses ACE-inhibiting properties. ACE is the enzyme which converts angiotensin I to angiotensin II. The ACE-inhibiting properties of an amide derivative of the invention may be demonstrated by its ability to prevent the cleavage of angiotensin I or of a synthetic peptide related to angiotensin I by ACE.

Angiotensin II is a potent constrictor of vascular smooth muscle, and is therefore involved in the control of blood pressure. A compound which prevents conversion of angiotensin I to angiotensin II will therefore lower circulating levels of angiotensin II and cause a fall in blood pressure. An amide derivative of the invention may therefore be used to lower blood pressure in a warm-blooded animal (including a human). At a dose of amide derivative of the invention which lowers blood pressure in an experimental animal, for example a rat, no symptoms of toxicity are apparent.

An amide derivative of the invention may be administered to a warm-blooded animal, including man, in the form of a pharmaceutical composition comprising as active ingredient at least one amide derivative of the invention, or a salt thereof, in association with a pharmaceutically-acceptable diluent or carrier therefor.

A suitable composition is, for example, a tablet, capsule, aqueous or oily solution or suspension, emulsion, injectable aqueous or oily solution or suspension, dispersible powder, spray or aerosol formulation.

The pharmaceutical composition may contain, in addition to the amide derivative of the invention, one or more drugs selected from diuretics, for example bendrofluazide, chlorothiazide and chlorthalidone; and other hypotensive agents, for example β-adrenergic blocking agents, for example atenolol and propranolol.

When used for the treatment of hypertension in man, it is expected that the amide derivative of the invention would be given to man at a total oral dose of between 1 mg. and 500 mg. daily, at doses spaced at 6—8 hourly intervals, or at an intravenous dose of between 0.1 mg. and 50 mg.

Preferred oral dosage forms are tablets or capsules containing between 1 mg. and 100 mg. of active ingredient. Preferred intravenous dosage forms are sterile aqueous solutions of active ingredient containing between 0.1% and 1% w/v of active ingredient.

The invention is illustrated but not limited by the following Examples:—

Example 1

A mixture of 2-thenoic acid (0.768 g.), thionyl chloride (10 ml.) and dimethylformamide (0.2 ml.) was heated under reflux for 90 minutes and the excess of thionyl chloride was then removed by evaporation under reduced pressure. The residue was dissolved in acetone (15 ml.) and the solution was added to a

stirred solution of *N* - [(R,S) - 5 - amino - (R,S) - 2 - methyl - 4 - oxo - 6 - phenylhexanoyl] - L - proline hydrochloride (1.3 g.) and sodium bicarbonate (0.9 g.) in water (25 ml.). The mixture was stirred at laboratory temperature for 16 hours, the acetone was removed by evaporation under reduced pressure and the aqueous residue was washed twice with ethyl acetate (50 ml. each time) and then acidified to pH 3 with aqueous 2N-hydrochloric acid. The mixture was extracted twice with ethyl acetate (50 ml each time) and the combined extracts were dried over magnesium sulphate and then evaporated to dryness under reduced pressure. The residue was purified by chromatography on a silica gel column (36 cm. long × 2 cm. diameter) using a 9:9:1:1 v/v/v/v mixture of toluene, ethyl acetate, methanol and formic acid as eluant. There was thus obtained as a gum *N* - [(R,S) - 2 - methyl - 4 - oxo - 6 - phenyl - (R,S) - 5 - (2 - thenoylamino) - hexanoyl] - L - proline, the structure of which was confirmed by elemental analysis, mass spectroscopy and proton magnetic resonance spectroscopy.

The *N* - [(R,S) - 5 - amino - (R,S) - 2 - methyl - 4 - oxo - 6 - phenylhexanoyl] - L - proline hydrochloride used as starting material was obtained as follows:—

A solution of 3-methoxycarbonylbutyric acid (29.2 g; J.Org.Chem., 1972, *37*, 555) and oxalyl chloride (31.5 g.) in benzene (100 ml.) was stirred at laboratory temperature for 4 hours, evaporated to dryness under reduced pressure and then twice re-evaporated to dryness from an excess of methylene chloride. A solution of the 3-methoxycarbonylbutyryl chloride thus obtained (20 g.) in dry tetrahydrofuran (40 ml.) was added dropwise during 15 minutes to a stirred solution of freshly distilled 4-benzyl-2-methyloxazol-5-(4H)-one (17.7 g.; prepared from phenylalanine and acetic anhydride as described in Annalen, 1926, *449*, 277), 4-dimethylamino-pyridine (0.7 g.) and triethylamine (27 ml.) in dry tetrahydrofuran (100 ml.) which had been cooled to −50°C., at such a rate that the temperature of the mixture did not exceed −30°C. The stirred mixture was allowed to warm up to laboratory temperature during 4 hours and was then stirred at laboratory temperature for 70 hours. Acetic acid (30 ml.) was added and the mixture was allowed to stand at laboratory temperature for 1 hour and then evaporated to dryness. The residue was partitioned between ethyl acetate (500 ml.) and aqueous 2N-hydrochloric acid (200 ml.), and the ethyl acetate layer was washed successively twice with aqueous 2N-hydrochloric acid, three times with 10% w/v aqueous sodium chloride solution, filtered through anhydrous sodium sulphate and evaporated to dryness under reduced pressure. The residue was purified by chromatography on a 30 cm. × 5 cm. silica gel column using a 1:1 v/v mixture of ethyl acetate and methylene chloride as eluant, and there was thus obtained methyl (R,S) - 5 - acetamido- (R,S) - 2 - methyl - 4 - oxo - 6 - phenylhexanoate (24.75 g.).

A mixture of methyl (R,S) - 5 - acetamido - (R,S) - 2 - methyl - 4 - oxo - 6 - phenylhexanoate (10.11 g.) and aqueous 6N-hydrochloric acid (100 ml.) was heated at 100°C. for 6 hours, cooled and evaporated to dryness under reduced pressure. Toluene (100 ml.) was added and the mixture again evaporated to dryness under reduced pressure. There was thus obtained as oily residue (R,S) - 5 - amino - (R,S) - 2 - methyl - 4 - oxo - 6 - phenylhexanoic acid hydrochloride. A suspension of sodium bicarbonate (10 g.) in water (50 ml.) was cautiously added to a stirred solution of the above hydrochloride (10.5 g.) in water (100 ml.), and after evolution of carbon dioxide has ceased a solution of benzyl chloroformate (6.1 ml.) in acetone (20 ml.) was added and the mixture was stirred at 5°C. for 18 hours. The mixture was washed with diethyl ether (200 ml.), acidified to pH 3 with aqueous 6N-hydrochloric acid, and extracted three times with ethyl acetate (50 ml. each time). The combined extracts were washed with water, dried over magnesium sulphate and evaporated to dryness under reduced pressure. There was thus obtained as oily residue (R,S) - 5 - benzyloxycarbonylamino - (R,S) - 2 - methyl - 4 - oxo - 6 - phenylhexanoic acid.

*N,N'*-Dicyclohexylcarbodi-imide (5.2 g.) was added to a stirred solution of (R,S) - 5 - benzyloxycarbonylamino - (R,S) - 2 - methyl - 4 - oxo - 6 - phenylhexanoic acid (9.5 g.), L-proline t-butyl ester (4.33 g.) and *N*-hydroxybenzotriazole (3.47 g.) in dry tetrahydrofuran (30 ml.) which was cooled to 0°C., and the mixture was allowed to warm up to laboratory temperature and was then stirred for 18 hours. The mixture was cooled to 0°C and filtered, and the filtrate was evaporated to dryness under reduced pressure. The residue was dissolved in ethyl acetate and the solution was washed with aqueous 10% w/v sodium bicarbonate solution and then with water, dried over magnesium sulphate and evaporated to dryness under reduced pressure. The residual oil was purified by chromatography on a silica gel column using a 1:1 v/v mixture of ethyl acetate and methylene chloride as eluant, and there was thus obtained as an oil *N* - [(R,S) - 5 - benzyloxycarbonylamino - (R,S) - 3 - methyl - 4 - oxo - 6 - phenylhexanoyl] - L - proline t-butyl ester.

A mixture of *N* - [(R,S) - 5 - benzyloxycarbonylamino - (R,S) - 2 - methyl - 4 - oxo - 6 - phenylhexanoyl]- L - proline t-butyl ester and a 9:1 v/v mixture of trifluoroacetic acid and water (25 ml.) was stirred at laboratory temperature for 1 hour, evaporated to dryness under reduced pressure and then re-evaporated to dryness from toluene and then from cyclohexane. The residue was purified by chromatography on silica gel using a 9:9:1 v/v/v/v mixture of ethyl acetate:toluene:formic acid:methanol as eluant. There was thus obtained *N* - [(R,S) - 5 - benzyloxycarbonylamino - (R,S) - 2 - methyl - 4 - oxo - 6 - phenylhexanoyl] - L - proline as an oil.

A 30% palladium-on-charcoal catalyst (1.0 g.) was suspended in a solution of *N* - (5 - benzyloxycarbonylamino - (R,S) - 2 - methyl - 4 - oxo - 6 - phenylhexanoyl) - L - proline (2.0 g.) in a mixture of ethanol (100 ml.) and concentrated hydrochloric acid (0.2 ml.), and the mixture was shaken under an atmosphere of hydrogen for 16 hours and then filtered. The filtrate was evaporated to dryness under reduced pressure, toluene (30 ml.) was added and the mixture was again evaporated to dryness under

reduced pressure, finally at 0.1 mm/Hg. There was thus obtained *N* - [(R,S) - 5 - amino - (R,S) - 2 - methyl - 4 - oxo - 6 - phenylhexanoyl] - L - proline hydrochloride sesquihydrate as a pale yellow solid, the structure of which was confirmed by elemental analysis.

Example 2

The process described in Example 1 was repeated except that *N* - [(S) - 5 - amino - 4 - oxo - 6 - phenylhexanoyl] - L - proline hydrochloride was used as starting material in place of the corresponding 2-methyl compound, and that a 2% by volume solution of acetic acid in ethyl acetate was used as eluant for the chromagraphic purification. There was thus obtained as a foam *N* - [4 - oxo - 6 - phenyl - (S) - 5 - (2 - thenoylamino)hexanoyl] - L - proline, the structure of which was confirmed by elemental analysis, mass spectroscopy and proton magnetic resonance spectroscopy.

The *N* - [(S) - 5 - amino - 4 - oxo - 6 - phenylhexanoyl] - L - proline hydrochloride used as starting material was obtained by a similar process to that described in the second part of Example 1, except that monomethyl succinate was used in place of 3-methoxycarbonylbutyric acid in the first stage. The (R,S) - 5 - benzyloxycarbonylamino - 4 - oxo - 6 - phenylhexanoic acid (corresponding to paragraph 3 of Example 1) was a crystalline solid, m.p. 107—109°C. The oily *N* - [(R,S) - 5 - benzyloxycarbonylamino - 4 - oxo - 6 - phenylhexanoyl] - L - proline t-butyl ester (corresponding to paragraph 4 of Example 1) was separated into its isomers by keeping a solution of 59 g. thereof in diethyl ether (300 ml.) at laboratory temperature for 18 hours and then filtering. The solid product was crystallised from a mixture of ethyl acetate and petroleum ether (b.p. 40—60°C.) and there was thus obtained *N* - [(S) - 5 - benzyloxycarbonylamino - 4 - oxo - 6 - phenylhexanoyl] - L - proline t-butyl ester, m.p. 126—128°C.

The diethyl ether mother liquors from the initial crystallisation were evaporated to dryness under reduced pressure. There was thus obtained as an oil *N* - [(R) - 5 - benzyloxycarbonylamino - 4 - oxo - 6 - phenylhexanoyl] - L - proline t-butyl ester.

Each isomer was then separately treated with trifluoroacetic acid to remove the t-butyl group, and then with hydrogen in the presence of a catalyst to remove the benzyloxycarbonyl group.

Example 3

The process described in Example 1 was repeated using the appropriate acid chloride and the appropriate 5-aminohexanoyl-L-proline hydrochloride as starting materials. There were thus obtained the compounds described in the following table, the structures of all of which were confirmed by elemental analysis and spectroscopic procedures:—

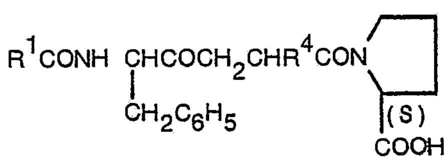

| $R^1$ | Isomer at 5-position | $R^4$ |
|---|---|---|
| 2-furyl | (R,S) | (R, S)-methyl |
| 2-furyl | (S) | H |
| 2-furyl | (R) | H |
| thien-2-ylmethyl | (S) | H |
| 2-pyridyl | (R) | H |

Example 4

*N*-Hydroxybenzotriazole (0.2295 g.) and then dicyclohexylcarbodi-imide (0.309 g.) were successively added to a stirred suspension of (R,S) - 5 - benzamido - 4 - oxo - 6 - (3 - thienyl)hexanoic acid (0.5175 g.) in a mixture of ethyl acetate (5 ml.) and methylene chloride (5 ml.), and the mixture was stirred at laboratory temperature for 90 minutes and then cooled to 0°C. L-Proline t-butyl ester (0.285 g.) was added and the mixture was stirred at laboratory temperature for 3 hours and then filtered. The filtrate was washed twice with saturated aqueous sodium carbonate solution and then with saturated aqueous sodium chloride solution, dried over sodium sulphate and evaporated to dryness under reduced pressure. The residue was dissolved in ethyl acetate, the mixture was filtered to remove solid dicyclohexylurea and the filtrate was

evaporated to dryness under reduced pressure. The residue was purified by chromatography on a silica gel column (31 cm. long × 1.25 cm. diameter) using a 1:1 v/v mixture of ethyl acetate and toluene as eluant. There was thus obtained as a gum *N* - [(R,S - 5 - benzamido - 4 - oxo - 6 - (3 - thienyl)hexanoyl] - L - proline t-butyl ester, the structure of which was confirmed by infra-red, proton magnetic resonance and mass spectroscopy.

The (R,S) - 5 - benzamido - 4 - oxo - 6 - (3 - thienyl) - hexanoic acid used as starting material was obtained as follows:—

A solution of 3-methoxycarbonylpropionyl chloride (27.1 ml.) in tetrahydrofuran (50 ml.) was added dropwise during 20 minutes to a stirred solution of 2-phenyloxazol-5(4H)-one (32.2 g.), triethylamine (61 ml.) and 4-dimethylaminopyridine (1.22 g.) in tetrahydrofuran (250 ml.) which was cooled to −50°C. and the mixture was stirred at −50°C. for a further 30 minutes and then poured into ice-water (1000 ml.). The mixture was acidified to pH 3 with aqueous 4N-hydrochloric acid and then filtered, and the solid was washed with water, dried and then crystallised from acetone. There was thus obtained 4 - (3 - methoxycarbonylpropionyl) - 2 - phenyloxazol - 5(4H) - one, m.p. 181—184°C. (with decomposition).

Triethylamine (5.52 ml.) and then 3-bromomethylthiophene (4.32 ml.) were successively added to a stirred suspension of the above oxazolone (10.96 g.) in dimethylformamide (45 ml.) and the mixture was stirred at laboratory temperature for 16 hours and then poured into water (400 ml.). The mixture was extracted three times with ethyl acetate (120 ml. each time) and the combined extracts were washed with water (120 ml.), dried and evaporated to dryness. A mixture of the residue, tetrahydrofuran (160 ml.), saturated aqueous sodium carbonate solution (80 ml.) and water (80 ml.) was stirred at laboratory temperature for 3.5 hours, diluted with water (80 ml.) and extracted four times with ethyl acetate (200 ml. each time). The combined extracts were washed twice with water (160 ml. each time) and once with saturated aqueous sodium chloride solution (160 ml.), dried and evaporated to dryness, and the residue was purified by chromatography on a silica gel column (39 cm. long × 5 cm. diameter) using a 5:1 v/v mixture of toluene and ethyl acetate as eluant. There was thus obtained methyl (R,S) - 5 - benzamido - 4 - oxo - 6 - (3 - thienyl) - hexanoate, m.p. 111—112°C.

A mixture of the above compound (0.69 g.), methanol (1.25 ml.) and aqueous 2N-sodium hydroxide solution (1.25 ml.) was stirred at laboratory temperature for 2.5 hours, the methanol was removed by evaporation and the residue was acidified to pH 2 with aqueous hydrochloric acid and then filtered. The solid residue was washed with water and dried, and there was thus obtained (R,S) - 5 - benzamido - 4 - oxo - 6 - (3 - thienyl)hexanoic acid, m.p. 169°C.

Example 5

The process described in Example 4 was repeated except that the appropriate 6-substituted-5-benzamido-4-oxohexanoic acid and the appropriate L-proline ester were used as starting materials, and there were thus obtained as gums the compounds described in the following table, the structures of all of which were confirmed by the spectroscopic techniques described in Example 4:—

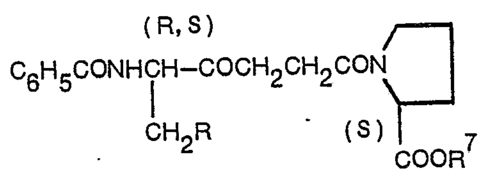

| R | $R^7$ |
|---|---|
| 5-methyl-2-furyl | methyl |
| 5-methyl-2-furyl | benzyl |
| 2-pyridyl | benzyl |
| 3-pyridyl | benzyl |
| imidazol-4-yl | benzyl |
| thiazol-4-yl | t-butyl |
| isoxazol-3-yl | t-butyl |
| benzimidazol-2-yl | benzyl |

The starting materials were prepared by a similar process to that described in the second part of Example 4, except that the appropriate bromomethyl-heterocycle (or chloromethyl-heterocycle plus one molar equivalent of sodium iodide) was used in place of 3-bromomethylthiophene. In the cases of the pyridyl, thiazolyl and imidazolyl compounds the sodium carbonate hydrolysis (third paragraph of Example 4) was unnecessary because the oxazolone was hydrolysed during the isolation process described in the second paragraph of Example 4.

In the cases of the pyridyl and imidazolyl compounds the final hexanoic acid was water soluble and was isolated by freeze-drying the final solution. It was therefore used as starting material contaminated with sodium chloride, which did not affect the proline coupling reaction.

## Example 6

A mixture of *N* - [(R,S) - 5 - benzamido - 4 - oxo - 6 - (3 - thienyl)hexanoyl] - L - proline t-butyl ester (Example 4; 0.2603 g.) and trifluoroacetic acid (2 ml.) was stirred at laboratory temperature for 2 hours, evaporated to dryness and then re-evaporated to dryness three times from toluene solution to remove all trace of trifluoroacetic acid. The residue was purified by chromatography on a silica gel column using acetone as eluant, and there was thus obtained as an oil *N* - [(R,S) - 5 - benzamido - 4 - oxo - 6 - (3 - thienyl)hexanoyl] - L - proline, the structure of which was confirmed by spectroscopic procedures.

The process described above was repeated using the appropriate t-butyl ester described in Example 5 as starting material. There were thus obtained, as gums, the compounds described in the following table, the structures of all of which were confirmed by spectroscopic procedures:

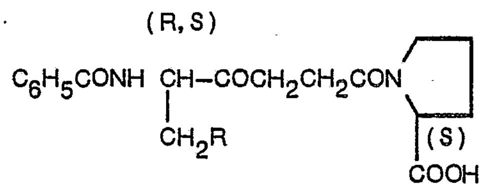

| R |
|---|
| thiazol-4-yl |
| isoxalol-4-yl |

## Example 7

A mixture of *N* - [(R,S) - 5 - benzamido - 4 - oxo - 6 - (3 - pyridyl)hexanoyl] - L - proline benzyl ester (Example 5; 0.1 g.), ethanol (10 ml.) and a 30% palladium-on-charcoal catalyst (0.0336 g.) was stirred under an atmosphere of hydrogen for 18 hours and then filtered. The filtrate was evaporated to dryness and there was thus obtained as residue, as a gum, *N* - [(R,S) - 5 - benzamido - 4 - oxo - 6 - (3 - pyridyl)hexanoyl] - L - proline, the structure of which was confirmed by spectroscopic procedures.

The process described above was repeated using the appropriate benzyl ester described in Example 4 as starting material. There were thus obtained, as gums, the compounds described in the following table, the structures of all of which were confirmed by spectroscopic procedures:—

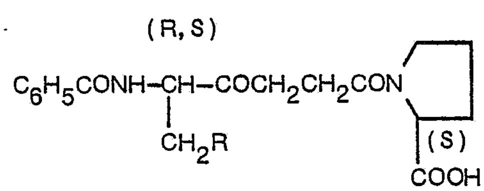

| R |
|---|
| 5-methyl-2-furyl |
| 2-pyridyl |
| imidazol-4-yl |
| benzimidazol-2-yl |

Example 8

A mixture of methanol (10 ml.) and thionyl chloride (1 ml.) was stirred at −40°C. for 15 minutes, *N* - [(R,S) - 2 - methyl - 4 - oxo - 6 - phenyl - (R,S) - 5 - (2 - thenoylamino)hexanoyl] - L - proline (Example 1; 0.3 g.) was added and the mixture was allowed to warm up to laboratory temperature, stirred at that temperature for 2 hours and then evaporated to dryness under reduced pressure. The residue was dissolved in ethyl acetate and the solution was washed twice with saturated aqueous sodium bicarbonate solution, dried over magnesium sulphate and evaporated to dryness under reduced pressure. There was thus obtained as an oil *N* - [(R,S) - 2 - methyl - 4 - oxo - 6 - phenyl - (R,S) - 5 - (2 - thenoylamino)hexanoyl] - L - proline methyl ester, the structure of which was confirmed by spectroscopic procedures.

Example 9

*N,N'*-Dicyclohexylcarbodiimide (0.46 g.) was added to a stirred solution of *N* - [(S] - 5 - (2 - furoylamino) - 4 - oxo - 6 - phenylhexanoyl] - L - proline (Example 3; 0.83 g.), 4-dimethylaminopyridine (0.1 g.) and isopropanol (0.3 ml.) in tetrahydrofuran (25 ml.) which was cooled to 0°C., and the mixture was stirred at laboratory temperature for 18 hours, recooled to 0°C. and filtered. The filtrate was evaporated to dryness under reduced pressure and the residue was purified by chromatography on a silica gel column using a 1:1 v/v mixture of methylene chloride and ethyl acetate as eluant. There was thus obtained, as a foam, *N* - [(S) - 5 - (2 - furoylamino) - 4 - oxo - 6 - phenylhexanoyl] - L - proline isopropyl ester, the structure of which was confirmed by elemental analysis and spectroscopic procedures.

The process described above was repeated except that *N* - [(R,S) - 5 - (2 - furoylamino) - (R,S) - 2 - methyl - 4 - oxo - 6 - phenylhexanoyl] - L - proline (Example 3) and methanol were used as starting materials. There was thus obtained *N* - [(R,S) - 5 - (2 - furoylamino) - (R,S) - 2 - methyl - 4 - oxo - 6 - phenylhexanoyl] - L - proline methyl ester, the structure of which was confirmed by elemental analysis and spectroscopic procedures.

Example 10

The process described in Example 4 was repeated using (R,S) - 5 - (2 - furoylamino - (R,S) - 2 - methyl - 4 - oxo - 6 - *p* - tolylhexanoic acid and proline t-butyl ester as starting materials. There was thus obtained, as an oil, *N* - [(R,S) - 5 - (2 - furoylamino) - (R,S) - 2 - methyl - 4 - oxo - 6 - *p* - tolylhexanoyl] - L - proline t-butyl ester, the structure of which was confirmed by spectroscopic procedures.

The (R,S) - 5 - (2 - furoylamino) - (R,S) - 2 - methyl - 4 - oxo - 6 - *p* - tolylhexanoic acid used as starting material was obtained as follows:—

Diethyl acetamidomalonate (108.5 g.) was slowly added to a stirred solution of sodium (12 g.) in ethanol (400 ml.) and the mixture was stirred at laboratory temperature for 30 minutes. Sodium iodide (5 g.) and *p*-methylbenzyl bromide (96 g.) were added and the mixture was stirred and heated under reflux for 16 hours, cooled and evaporated to dryness under reduced pressure. The residue was partitioned between water (250 ml.) and ethyl acetate (500 ml.) and the organic layer was washed with saturated aqueous sodium chloride solution, dried over magnesium sulphate and evaporated to dryness. The residue was stirred with a 4:1 v/v mixture of petroleum ether (b.p. 40—60°C.) and diethyl ether and the mixture was filtered. There was thus obtained ethyl 2 - acetamido - 2 - ethoxycarbonyl - 3 - *p* - tolylpropionate, m.p. 111—113°C.

A stirred suspension of the above diester (127 g.) in 10% aqueous sodium hydroxide solution (600 ml.) was heated under reflux for 4 hours, cooled, acidified with aqueous 3N-hydrochloric acid, reheated under reflux for 1 hour, cooled to 0°C., kept at that temperature for 72 hours and then filtered. There was thus obtained as solid residue (±) - 2 - acetamido - 3 - *p* - tolypropionic acid.

A mixture of the above acid (35 g.) and acetic anhydride (300 ml.) was stirred at 100°C for 40 minutes, cooled and the excess of acetic anhydride was removed by evaporation under reduced pressure. The residue was distilled and there was thus obtained 2 - methyl - 4 - *p* - tolyloxazol - 5 - (4H) - one, b.p. 116—120°C/0.2 mm.Hg.

This oxazolone was reacted with 3-methoxycarbonylbutyryl chloride by a similar process to that described in the second part of Example 1 to give methyl (R,S) - 5 - acetamido - (R,S) - 2 - methyl - 4 - oxo - 6 - *p* - tolylhexanoate; this was hydrolysed with aqueous 6N hydrochloric acid by a similar process to that described in the third part of Example 1; and the product was acylated with 2-furoyl chloride by a similar procedure to that described in the first part of Example 1. There was thus obtained (R,S) - 5 - (2 - furoylamino - (R,S) - 2 - methyl - 4 - oxo - 6 - *p* - tolylhexanoic acid which was used without further purification.

Example 11

The process described in Example 6 was repeated using the t-butyl ester of Example 10 as starting material. There was thus obtained, as an oil, *N*- [(R,S) - 5 - (2 - furoylamino) - (R,S) - 2 - methyl - 4 - oxo - 6 - *p* - tolylhexanoyl] - L - proline, the structure of which was confirmed by spectroscopic procedures.

**Claims**

1. An amide derivative of the formula:—

$$R^1—Y—NR^2—CHR^3—X—CHR^{11}—CHR^4—CO—NR^5—CR^6R^{16}—Q—R^{10}$$

wherein either $R^1$ is hydrogen, or alkyl of up to 15 carbon atoms which is unsubstituted, or which bears one substituent selected from hydroxy, alkoxy, alkylthio and alkoxycarbonyl each of up to 5 carbon atoms, and aryl, aryloxy, arylthio and heterocyclic substituents; or which bears two or three substituents one of which is aryl, another of which is hydroxy, amino, trifluoromethyl, aryloxy or alkoxy of up to 5 carbon atoms and the third of which, if present, is aryl or trifluoromethyl;

or $R^1$ is aryl or, when Y is carbonyl or thiocarbonyl, is aryloxy, alkoxy or arylalkoxy wherein the alkoxy part has up to 5 carbon atoms;

or $R^1$ is halogenoalkyl of up to 6 carbon atoms or is alkenyl, or cycloalkyl each of up to 6 carbon atoms which is unsubstituted or which bears an aryl substituent;

or $R^1$ is a substituent of the formula $R^8CONHCHR^9—$ or $R^8COCH_2CHR^9—$ wherein $R^8$ is alkyl or cycloalkyl each of up to 10 carbon atoms, or aryl and $R^9$ is hydrogen, or alkyl of up to 5 carbon atoms which is unsubstituted or which bears an aryl substituent, or $R^9$ is a group other than those stated above such that the compound $H_2NCHR^9COOH$ would be a naturally-occurring amino acid;

or $R^1$ is heterocyclic;

wherein $R^2$ is hydrogen, alkyl of up to 5 carbon atoms which is unsubstituted or which bears an aryl substituent, or aryl;

wherein $R^3$ is alkyl or alkenyl each of up to 5 carbon atoms which is unsubstituted or which bears an aryl substituent;

or $R^3$ is aryl or indolylmethyl or $R^3$ is alkyl of up to 5 carbon atoms which is substituted by heterocyclic, other than methyl substituted by indolyl;

provided that when $R^1$ is other than heterocyclic or alkyl substituted by heterocyclic, $R^3$ must be alkyl substituted by heterocyclic other than methyl substituted by indolyl.

wherein $R^4$ is hydrogen or alkyl of up to 5 carbon atoms which is unsubstituted or which bears an aryl substituent;

wherein either $R^5$ is hydrogen or aryl, or alkyl of up to 5 carbon atoms which is unsubstituted or which bears an aryl substituent;

or $R^5$ is joined together with $R^6$ as defined below;

wherein either $R^6$ is hydrogen, aryl or heterocyclic, or alkyl of up to 5 carbon atoms which is unsubstituted or which bears a hydroxy, aryl or heterocyclic substituent;

or $R^6$ and $R^5$ are joined together to form alkylene or alkenylene of 2 to 5 carbon atoms; or an oxa-, thia- or aza-derivative of said alkylene or alkenylene; or an oxo-substituted derivative of said alkylene or alkyenylene;

or $R^6$ and $R^{16}$, or $R^6$, $R^{16}$ and $R^5$, or $R^6$ and $R^{10}$ are joined together as defined below;

wherein $R^{16}$ is hydrogen or alkyl of up to 5 carbon atoms;

or $R^6$ and $R^{16}$ are joined together to form alkylene of 2 to 5 carbon atoms (that is, to form a spirocycloalkyl group);

or $R^{16}$ together with the first carbon atom of $R^6$ form a double bond wherein $R^6$ is otherwise alkyl, or wherein $R^6$ is otherwise substituted alkyl as defined above, or wherein $R^6$ and $R^5$ are otherwise joined together as defined above (that is, so that $R^5$, $R^6$ and $R^{16}$ together form alkylidene);

wherein Q is carbonyl (—CO—) or methylene ($—CH_2—$);

and wherein either $R^{10}$ is hydroxy, amino, aryloxy, arylthio, alkoxy, cycloalkoxy, alkylamino, dialkylamino, cyclic amino, hydroxyalkoxy, acyloxyalkoxy, aminoalkoxy, alkylaminoalkoxy, dialkylaminoalkoxy, cyclic aminoalkoxy or arylalkoxy wherein each alkyl or alkoxy has up to 5 carbon atoms and wherein cyclic amino has up to 6 carbon atoms;

or wherein $R^{10}$ and $R^6$ are joined together such that $R^{10}$ is oxygen (—O—) joined to the terminal carbon atom of $R^6$ when it is alkyl;

wherein $R^{11}$ is hydrogen or alkyl of up to 3 carbon atoms;

wherein X is carbonyl (—CO—), hydroxymethylene (—CHOH—), thiocarbonyl (—CS—) or oximinomethylene ($—C{=}N—OR^{20}$, wherein $R^{20}$ is hydrogen or alkyl of up to 5 carbon atoms);

and wherein Y is carbonyl, thiocarbonyl, sulphonyl ($—SO_2—$) or amido (—NHCO—);

or a salt thereof where appropriate.

2. An amide derivative as claimed in claim 1 wherein X, Y and Q are all carbonyl and $R^2$ and $R^{11}$ are both hydrogen, or a salt thereof.

3. An amide derivative of the formula:—

$$R^1CONH—CHR^3—COCH_2—CHR^4—CONR^5—CHR^6—COR^{10}$$

wherein $R^1$ is methyl, benzyl, β-phenylethyl, benzyloxy, phenoxymethyl, phenylthiomethyl, phenyl, tolyl, methoxyphenyl, methylthiophenyl, monochlorophenyl, dichlorophenyl, fluorophenyl, iodophenyl,

nitrophenyl, cyanophenyl, trifluoromethylphenyl, acetamidophenyl, acetylphenyl, methanesulphonylphenyl, biphenylyl, naphthyl, benzamidomethyl, cyclopentanecarbonamidomethyl or β-benzoylethyl;

$R^3$ is methyl substituted by a 5- or 6-membered, fully-unsaturated heterocyclic ring containing 1 or 2 hetero- atoms selected from oxygen, nitrogen and sulphur, which ring may optionally contain a methyl substituent or be fused to a benzene ring, provided that $R^3$ is not indolyl-methyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-thiatrimethylene, 3-thiatetramethylene, 3-oxatetramethylene, 3-methyl-3-azatetramethylene; and $R^{10}$ is hydroxy, amino, alkoxy of up to 5 carbon atoms, β-dimethylaminoethoxy or β-morpholinoethoxy, or benzyloxy, or a salt thereof.

4. An amide derivative as claimed in claim 3 wherein $R^1$ is phenyl, $R^3$ is methyl substituted by 3-thienyl, 5-methyl-2-furyl, 2-pyridyl, 3-pyridyl, 4-thiazolyl, 4-imidazolyl, 3-isoxazolyl or 2-benzimidazolyl, $R^4$ is hydrogen or methyl, $R^5$ and $R^6$ together form trimethylene and $R^{10}$ is hydroxy, alkoxy of up to 5 carbon atoms or benzyloxy, or a salt thereof.

5. An amide derivative of the formula:—

$$R^1CONH—CHR^3—COCH_2—CHR^4—CONR^5—CHR^6—COR^{10}$$

wherein $R^1$ is a 5- or 6-membered, fully-unsaturated heterocyclic ring containing 1 or 2 hetero-atoms selected from oxygen, nitrogen and sulphur, which ring may optionally contain a methyl substituent or be fused to a benzene ring;

$R^3$ is benzyl which is unsubstituted or bears a methyl, t-butyl, methoxy, fluoro, chloro, bromo, nitro, cyano, trifluoromethyl, amino, acetamido, methanesulphonamido, trifluoromethanesulphonamido or phenyl substituent, or

$R^3$ is 3-phenylpropyl or 3-phenylprop-2-enyl;

$R^4$ is hydrogen or methyl;

$R^5$ is hydrogen or methyl and $R^6$ is hydrogen, benzyl or indol-3-ylmethyl, or $R^5$ and $R^6$ together form trimethylene, tetramethylene, pentamethylene, 2-thiatrimethylene, 3-thiatetramethylene, 3-oxatetramethylene or 3-methyl-3-azatetramethylene; and

$R^{10}$ is hydroxy, amino, alkoxy of up to 5 carbon atoms, β-dimethylaminoethoxy, β-morpholinoethoxy or benzyloxy, or a salt thereof.

6. An amide derivative as claimed in claim 5 wherein $R^1$ is 2-furyl, 2-thienyl or 2-pyridyl, $R^3$ is benzyl, $R^4$ is hydrogen or methyl, $R^5$ and $R^6$ together form trimethylene and $R^{10}$ is hydroxy or alkoxy of up to 5 carbon atoms, or a salt thereof.

7. The compound *N* - [2 - methyl - 4 - oxo - 6 - phenyl - 5 - (2 - thenoylamino)hexanoyl] - L - proline; *N* - [5 - (2 - furoylamino) - 4 - oxo - 6 - phenylhexanoyl] - L - proline isobutyl ester; *N* - [5 - benzamido - 4 - oxo - 6 - (pyrid - 3 - yl)hexanoyl] - L - proline or *N* - [5 - benzamido - 6 - (imidazol - 4 - yl) - 4 - oxohexanoyl] - L - proline.

8. A salt of an amide derivative, claimed in any of claims 1 to 7 wherein $R^{10}$ is hydroxy, which is an alkali metal or alkaline earth metal salt, or an ammonium or dicyclohexylamine salt.

9. A process for the manufacture of an amide derivative or a salt thereof, claimed in any of claims 1 to 8, which comprises the reaction of an acid of the formula:—

$$R^1—Y—NR^2—CHR^3—X—CHR^{11}—CHR^4—COOH$$

wherein $R^1$, $R^2$, $R^3$, $R^4$, $R^{11}$, X and Y have the meanings stated in any of claims 1 to 5, or of a reactive derivative thereof, with an amine of the formula:—

$$HNR^5—CR^6R^{16}—Q—R^{12}$$

wherein $R^5$, $R^6$, $R^{16}$ and Q have the meanings stated in any of claims 1 to 5 and wherein $R^{12}$ has any of the meanings stated in claims 1 to 5 for $R^{10}$ except those containing a free hydroxy or amino group whereafter an amide derivative wherein $R^2$ is alkyl, arylalkyl or aryl (and $R^{10}$ is $R^{12}$) may be obtained by reacting the corresponding amide derivative wherein $R^2$ is hydrogen (and $R^{10}$ is $R^{12}$) with a compound of the formula $R^2Z$, wherein $R^2$ has the meaning stated above and Z is a displaceable halogen, alkanesulphonyl or arenesulphonyl group;

or whereafter an amide derivative wherein Q is carbonyl and $R^{10}$ is hydroxy may be obtained by the hydrolysis of the corresponding amide derivative wherein $R^{10}$ is alkoxy, or, when $R^{10}$ is t-butoxy, by the acid-catalysed cleavage of said compound;

or whereafter an amide derivative wherein X is hydroxymethylene may be obtained by the reduction of the corresponding amide derivative wherein X is carbonyl; or whereafter an amide derivative wherein X is oximinomethylene may be obtained by the reaction of the corresponding amide derivative wherein X is carbonyl with hydroxylamine or an *O*-substituted hydroxylamine derivative.

10. A pharmaceutical composition comprising as active ingredient at least one amide derivative, or a

salt thereof, claimed in any of claims 1 to 8, in association with a pharmaceutically-acceptable diluent or carrier therefor.

**Patentansprüche**

1. Amidderivat der Formel

$$R^1—Y—NR^2—CHR_3—X—CHR^{11}—CHR^4—CO—NR^5—CR^6R^{16}—Q—R^{10}$$

worin $R^1$ entweder für Wasserstoff oder Alkyl mit bis zu 15 Kohlenstoffatomen steht, das unsubstituiert ist oder das einen Substituenten trägt, der aus Hydroxy, Alkoxy, Alkylthio und Alkoxycarbonyl mit jeweils bis zu 5 Kohlenstoffatomen und Aryl, Aryloxy, Arylthio und heterocyclischen Substituenten ausgewählt ist, oder das zwei oder drei Substituenten trägt, von denen einer Aryl ist, von denen ein weiterer Hydroxy, Amino, Trifluoromethyl, Aryloxy oder Alkoxy mit bis zu 5 Kohlenstoffatomen ist und von denen der dritte, sofern anwesend, Aryl oder Trifluoromethyl ist; oder $R^1$ für Aryl oder, sofern Y Carbonyl oder Thiocarbonyl ist, für Aryloxy, Alkoxy oder Arylalkoxy steht, worin der Alkoxyteil bis zu 5 Kohlenstoffatome aufweist; oder $R^1$ für Halogenalkyl mit bis zu 6 Kohlenstoffatomen oder für Alkenyl oder Cycloalkyl mit jeweils bis zu 6 Kohlenstoffatomen, das unsubstituiert ist oder das einen Arylsubstituenten trägt, steht; oder $R^1$ für einen Substituenten der Formel $R^8CONHCHR^9—$ oder $R^8COCH_2CHR^9—$ steht, worin $R^8$ Alkyl oder Cycloalkyl mit jeweils bis zu 10 Kohlenstoffatomen oder Aryl ist und $R^9$ Wasserstoff oder Alkyl mit bis zu 5 Kohlenstoffatomen ist, das unsubstituiert ist oder das einen Arylsubstituenten trägt, oder $R^9$ eine andere Gruppe als eine der obengenannten ist, so daß die Verbindung $H_2NCHR^9COOH$ eine natürlich vorkommende Aminosäure wäre; oder $r^1$ für einen Heterocyclus steht; worin $R^2$ für Wasserstoff, Alkyl mit bis zu 5 Kohlenstoffatomen, das unsubstituiert ist oder das einen Arylsubstituenten trägt, oder Aryl steht; worin $R^3$ für Alkyl oder Alkenyl mit jeweils bis zu 5 Kohlenstoffatomen steht, das unsubstituiert ist oder das einen Arylsubstituenten trägt; oder $R^3$ für Aryl oder Indolylmethyl steht oder $R^3$ für Alkyl mit bis zu 5 Kohlenstoffatomen, das durch einen Heterocyclus substituiert ist, jedoch mit Ausnahme von Methyl, das durch Indolyl substituiert ist, steht; mit der Maßgabe, daß, wenn $R^1$ für etwas anderes als einen Heterocyclus oder Alkyl, das durch einen Heterocyclus substituiert ist, steht, $R^3$ für Alkyl, das durch einen Heterocyclus substituiert ist, mit Ausnahme von Methyl, das durch Indolyl substituiert ist, stehen muß; worin $R^4$ für Wasserstoff oder Alkyl mit bis zu 5 Kohlenstoffatomen steht, das unsubstituiert ist oder das einen arylsubstituenten trägt; worin $R^5$ entweder für Wasserstoff oder Aryl oder Alkyl mit bis zu 5 Kohlenstoffatomen, das unsubstituiert ist oder das einen Arylsubstituenten trägt, steht; oder $R^5$ mit $R^6$ verbunden ist, wie es weiter unten definiert ist; worin $R^6$ entweder für Wasserstoff, Aryl oder einen Heterocyclus oder Alkyl mit bis zu 5 Kohlenstoffatomen, das unstubstiituiert ist oder das einen Hydroxy-, Aryl- oder heterocyclischen Substituenten trägt, steht; oder $R^5$ und $R^6$ miteinander verbunden sind, so daß Alkylen oder Alkenylen mit 2 bis 5 Kohlenstoffatomen oder ein Oxa-, Thia- oder Azaderivat dieses Alkylens oder Alkenylens oder ein oxosubstituiertes Derivat dieses Alkylens oder Alkenylens gebildet wird; oder $R^6$ und $R^{16}$ oder $R^6$, $R^{16}$ und $R^5$ oder $R^6$ und $R^{10}$ miteinander verbunden sind, wie es weiter unten definiert ist, worin $R^{16}$ für Wasserstoff oder Alkyl mit bis zu 5 Kohlenstoffatomen steht; oder $R^6$ und $R^{16}$ miteinander verbunden sind, so daß Alkylen mit 2 bis 5 Kohlenstoffatomen gebildet wird (das heißt, daß eine Spirocycloalkylgruppe gebildet wird); oder $R^{16}$ zusammen mit dem ersten Kohlenstoffatom von $R^6$ eine Doppelbindung bildet, wobei $R^6$ ansonsten Alkyl ist oder Wobei $R^6$ ansonsten substituiertes Alkyl ist, wie es oben definiert ist, oder wobei $R^6$ und $R^5$ ansonsten miteinander verbunden sind, wie es oben definiert ist (das heißt, daß $R^5$, $R^6$ und $R^{16}$ gemeinsam Alkyliden bilden); worin Q für Carbonyl (—CO—) oder Methylen (—$CH_2$—) steht; worin $R^{10}$ entweder für Hydroxy, Amino, Aryloxy, Arylthio, Alkoxy, Cycloalkoxy, Alkylamino, Dialkylamino, cyclisches Amino, Hydroxyalkoxy, Acyloxyalkoxy, Aminoalkoxy, Alkylaminoalkoxy, Dialkylaminoalkoxy, cyclisches Aminoalkoxy oder Arylalkoxy steht, worin jedes Alkyl oder Alkoxy bis zu 5 Kohlenstoffatome aufweist und worin cyclisches Amino bis zu 6 Kohlenstoffatome aufweist; oder worin $R^{10}$ und $R^6$ miteinander verbunden sind, so daß $R^{10}$ Sauerstoff (—O—) ist, der mit dem endständigen Kohlenstoffatom von $R^6$ verbunden ist, wenn dieses Alkyl ist; worin $R^{11}$ für Wasserstoff oder Alkyl mit bis zu 3 Kohlenstoffatomen steht; worin X für Carbonyl (—CO—), Hydroxymethylen (—CHOH—), Thiocarbonyl (—CS—) oder Oximinomethylen (—C=N—$OR^{20}$, worin $R^{20}$ Wasserstoff oder Alkyl mit bis zu 5 Kohlenstoffatomen ist) steht; und worin Y für Carbonyl, Thiocarbonyl, Sulfonyl (—$SO_2$—) oder Amino (—NHCO—) steht; oder ein Salz davon, sofern möglich.

2. Amidderivat nach Anspruch 1, worin X, Y und Q alle für Carbonyl stehen und $R^2$ und $R^{11}$ beide für Wasserstoff stehen, oder ein Salz davon.

3. Amidderivat der Formel

$$R^1CONH—CHR^3—COCH_2—CHR^4—CONR^5—CHR^6—COR^{10}$$

worin $R^1$ für Methyl, Benzyl, β-Phenyläthyl, Benzyloxy, Phenoxymethyl, Phenylthiomethyl, Phenyl, Tolyl, Methoxyphenyl, Methylthiophenyl, Monochlorophenyl, Dichlorophenyl, Fluorophenyl, Jodophenyl, Nitrophenyl, Cyanophenyl, Trifluoromethylphenyl, Acetamidophenyl, Acetylphenyl, Methansulfonylphenyl, Biphenylyl, Naphthyl, Benzamidomethyl, Cyclopentancarbonamidomethyl oder β-

Benzoyläthyl steht; $R^3$ für Methyl steht, das durch einen 5- oder 6 gliedrigen, vollständig ungesättigten heterocyclischen Ring mit 1 oder 2 Heteroatomen substituiert ist, die aus Sauerstoff, Stickstoff und Schwefel ausgewählt sind, wobei der Ring gegebenenfalls einen Methylsubstituierten enthalten oder mit einem Benzolring kondensiert sein kann, mit der Maßgabe, daß $R^3$ nicht für Indolylmethyl steht; $R^4$, für Wasserstoff oder Methyl steht; $R^5$ für Wasserstoff oder Methyl steht und $R^6$ für Wasserstoff, Benzyl oder Indol-3-ylmethyl steht oder $R^5$ und $R^6$ gemeinsam Trimethylen, Tetramethylen, Pentamethylen, 2-Thiatrimethylen, 3-Thiatetramethylen, 3-Oxatetramethylen oder 3-Methyl-3-azatetramethylen bilden; und $R^{10}$ für Hydroxy, Amino, Alkoxy mit bis zu 5 Kohlenstoffatomen, β-Dimethylaminoäthoxy, β-Morpholinoäthoxy oder Benzyloxy steht; oder ein Salz davon.

4. Amidderivat nach Anspruch 3, worin $R^1$ für Phenyl steht, $R^3$ für Methyl steht, das durch 3-Thienyl, 5-Methyl-2-furyl, 2-Pyridyl, 3-Pyridyl, 4-Thiazolyl, 4-Imidazolyl, 3-Isoxazolyl oder 2-Benzimidazolyl substituiert ist, $R^4$ für Wasserstoff oder Methyl steht, $R^5$ und $R^6$ gemeinsam Trimethylenbilden und $R^{10}$ für Hydroxy, Alkoxy mit bis zu 5 Kohlenstoffatomen oder Benzyloxy steht, oder ein Salz davon.

5. Amidderivat der Formel

$$R^1CONH—CHR^3—COCH_2—CHR^4—CONR^5—CHR^6—COR^{10}$$

worin $R^1$ für einen 5- oder 6 gliedrigen, vollständig ungesättigten heterocyclischen Ring mit 1 oder 2 Heteroatomen steht, die aus Sauerstoff, Stickstoff und Schwefel ausgewählt sind, wobei der Ring gegebenenfalls einen Methylsubstituenten enthalten oder mit einem Benzolring kondensiert sein kann; $R^3$ für Benzyl steht, das unsubstituiert ist oder das einen Methyl-, t-Butyl-, Methoxy-, Fluoro-, Chloro-, Bromo-, Nitro-, Cyano-, Trifluoromethyl-, Amino-, Acetamido-, Methansulfonamido, Trifluoromethansulfonamido- oder Phenylsubstituenten trägt, oder $R^3$ für 3-Phenylpropyl oder 3-Phenylprop-2-enyl steht; $R^4$ für Wasserstoff oder Methyl steht; $R^5$ für Wasserstoff oder Methyl steht und $R^6$ für Wasserstoff, Benzyl oder Indol-3-ylmethyl steht oder $R^5$ und $R^6$ gemeinsam Trimethylen, Tetramethylen, Pentamethylen, 2-Thiatrimethylen, 3-Thiatetramethylen, 3-Oxatetramethylen oder 3-Methyl-3-azatetramethylen bilden; und $R^{10}$ für Hydroxy, Amino, Alkoxy mit bis zu 5 Kohlenstoffatomen, β-Dimethylaminoäthoxy, β-Morpholinoäthoxy oder Benzyloxy steht; oder ein Salz davon.

6. Amidderivat nach Anspruch 5, worin $R^1$ für 2-Furyl, 2-Thienyl oder 2-Pyridyl steht, $R^3$ für Benzyl steht, $R^4$ für Wasserstoff oder Methyl steht, $R^5$ und $R^6$ gemeinsam Trimethylen bilden und $R^{10}$ für Hydroxy oder Alkoxy mit bis zu 5 Kohlenstoffatomen steht, oder ein Salz davon.

7. Die Verbindung N-[2-Methyl-4-oxo-6-phenyl-5-(2-thenoyl-amino)hexanoyl]-L-prolin, N-[5-(2-Furoylamino)-4-oxo-6-phenylhexanoyl]-L-prolin-isobutylester, N-[5-Benzamido-4-oxo-6-(pyrid-3-yl)hexanoyl]-L-prolin oder N-[5-Benzamido-6-(imidazol-4-yl)-4-oxohexanoyl]-L-prolin.

8. Ein Salz eines Amidderivats nach einem der Ansprüche 1 bis 7, worin $R^{10}$ für Hydroxy steht, bei welchem es sich um ein Alkalimetall- oder Erdalkalimetallsalz oder um ein Ammonium- oder Dicychlohexylaminsalz handelt.

9. Verfahren zur Herstellung eines Amidderivats oder eines Salzes davon, wie sie in einem der Ansprüche 1 bis 8 beansprucht werden, bei welchem eine Säure der Formel

$$R^1—Y—NR^2—CHR^3—X—CHR^{11}—CHR^4—COOH$$

worin $R^1$, $R^2$, $R^3$, $R^4$, $R^{11}$, X und Y die in einem der Ansprüche bis 5 angegebenen Bedeutungen besitzen, oder ein reaktives Derivat davon mit einem Amin der Formel

$$HNR^5—CH^6R^{16}—Q—R^{12}$$

worin $R^5$, $R^6$, $R^{16}$ und Q die in einem der Ansprüche 1 bis 5 angegebenen Bedeutungen besitzen und worin $R^{12}$ eine der für $R^{10}$ in einem der Ansprüche 1 bis 5 angegebene Bedeutung besitzt, mit Ausnahme solcher, die eine freie Hydroxy-oder Aminogruppe enthalten, umgesetzt worauf wird; ein Amidderivat, worin $R^2$ für Alkyl, Arylalkyl oder Aryl steht (und $R^{10}$ gleich $R^{12}$ ist) erhalten werden kann, indem man das entsprechende Amidderivat, worin $R^2$ für Wasserstoff steht (und $R^{10}$ gleich $R^{12}$ ist), mit einer Verbindung der Formel $R^2Z$, worin $R^2$ die oben angegebene Bedeutung besitzt und Z für ersetzbares Halogen oder eine ersetzbare Alkansulfonyl- oder Arensulfonylgruppe steht, umsetzt; oder worauf ein Amidderivat, worin Q für Carbonyl steht und $R^{10}$ für Hydroxy steht, erhalten werden kann, indem man das entsprechende Amidderivat, worin $R^{10}$ für Alkoxy steht, hydrolisiert oder indem man, wenn $R^{10}$ für t-Butoxy steht, diese Verbindung einer sauer katalysierten Spaltung unterwirft; oder worauf ein Amidderivat, worin X für Hydroxymethylen steht, erhalten werden kann, indem man das entsprechende Amidderivat, worin X für Carbonyl steht, reduziert; oder worauf ein Amidderivat, worin X für Oximinomethylen steht, erhalten werden kann, indem man das entsprechende Amidderivat, worin X für Carbonyl steht, mit Hydroxylamin oder einem O-substituierten Hydroxylaminderivat umsetzt.

10. Pharmazeutische Zusammensetzung, welche als aktiven Bestandteil mindestens ein Amidderivat oder ein Salz davon, wie sie in einem der Ansprüche 1 bis 8 beansprucht werden, gemeinsam mit einem pharmazeutisch zulässigen Verdünnungsmittel oder Trägermittel hierfür enthält.

**Revendications**

1. Dérivé amidique de formule:

$$R^1—Y—NR^2—CHR_3—X—CHR^{11}—CHR^4—CO—NR^5—CR^6R^{16}—Q—R^{10}$$

dans laquelle $R^1$ est l'hydrogène ou un groupe alkyle ayant jusqu'à 15 atomes de carbone qui n'est pas substitué ou qui porte un substituant choisi entre des substituants hydroxy, alkoxy, alkylthio et alkoxycarbonyle ayant chacun jusqu'à 5 atomes de carbone, et des substituants aryle, aryloxy, arylthio et hétérocycliques; ou qui porte deux ou trois substituants dont l'un est un groupe aryle, un autre est un groupe hydroxy, amino, trifluorométhyle, aryloxy ou alkoxy ayant jusqu'à 5 atomes de carbone et le troisième, s'il est présent, est un groupe aryle ou trifluoro--méthyle; ou bien $R^1$ est un groupe aryle ou, lorsque Y est un groupe carbonyle ou thiocarbonyle, $R^1$ est un groupe aryloxy, alkoxy ou arylalkoxy dont la portion alkoxy comprend jusqu'à 5 atomes de carbone; ou bien $R^1$ est un groupe halogénalkyle ayant jusqu'à 6 atomes de carbone ou un groupe alcényle ou cycloalkyle ayant chacun jusqu'à 6 atomes de carbone, qui n'est pas substitué ou qui porte un substituant aryle; ou bien $R^1$ est un substituant de formule $R^8CONHCHR^9—$ ou $R^8COCH_2CHR^9—$ dans laquelle $R^8$ est un radical alkyle ou cycloalkyle ayant chacun jusqu'à 10 atomes de carbone ou un radical aryle, et $R^9$ est l'hydrogène ou un radical alkyle ayant jusqu'à 5 atomes de carbone qui n'est pas substitué ou qui porte un substituant aryle, ou bien $R^9$ est un groupe autre que ceux qui sont indiqués ci-dessus, de telle sorte que le composé $H_2NCHR^9COOH$ représente un amino-acide naturel; ou bien $R^1$ est un groupe hétérocyclique; $R^2$ est l'hydrogène, un groupe alkyle ayant jusqu'à 5 atomes de carbone qui n'est pas substitué ou qui porte un substituant aryle, ou un groupe aryle; $R^3$ est un groupe alkyle ou alcényle, chacun ayant jusqu'à 5 atomes de carbone, qui n'est pas substitué ou qui porte un substituant aryle; ou bien $R^3$ est un groupe aryle ou indolylméthyle ou représente un groupe alkyle ayant jusqu'à 5 atomes de carbone qui est substitué par un radical hétérocyclique, autre qu'un groupe méthyle substitué par un radical indolyle; sous réserve que lorsque $R^1$ représente autre chose qu'un groupe hétérocyclique ou qu'un groupe alkyle substitué par un radical hétérocyclique, $R^3$ doive être un groupe alkyle substitué par un radical hétérocyclique autre qu'un groupe méthyle substitué par un radical indolyle, $R^4$ est l'hydrogène ou un groupe alkyle ayant jusqu'à 5 atomes de carbone qui n'est pas substitué ou qui porte un substituant aryle; $R^5$ est l'hydrogène ou un groupe aryle, ou représente un groupe alkyle ayant jusqu'à 5 atomes de carbone qui n'est pas substitué ou qui porte un substituant aryle; ou bien $R^5$ s'associe avec $R^6$ comme défini ci-dessous; $R^6$ est l'hydrogène, un groupe aryle ou hétérocyclique, ou un groupe alkyle ayant jusqu'à 5 atomes de carbone qui n'est pas substitué ou qui porte un substituant hydroxy, aryle ou hétérocyclique; ou bien $R^6$ et $R^5$ forment conjointement un groupe alkylène ou alcénylène de 2 à 5 atomes de carbone; ou un dérivé oxa, thia ou aza dudit alkylène ou alcénylène; ou un dérivé à substituant oxo dudit alkylène ou alcénylène; ou bien $R^6$ et $R^{16}$, ou $R^6$, $R^{16}$ et $R^5$, ou $R^6$ et $R^{10}$ s'associent comme défini ci-dessous; $R^{16}$ est l'hydrogène ou un groupe alkyle ayant jusqu'à 5 atomes de carbone; ou bien $R^6$ et $R^{16}$ forment conjointement un groupe alkylène de 2 à 5 atomes de carbone (c'est-à-dire qu'ils s'associent pour former un groupe spirocycloalkyle); ou bien $R^{16}$ forme en association avec le premier atome de carbone de $R^6$ une double liaison dans laquelle $R^6$ représente autre chose qu'un grope alkyle, ou bien $R^6$ est un groupe alkyle autrement substitué tel que défini ci-dessus, ou bien $R^6$ et $R^5$ s'associent d'une autre façon comme défini ci-dessus (c'est-à-dire de manière que $R^5$, $R^6$ et $R^{16}$ forment ensemble un groupe alkylidène); Q est un groupe carbonyle (—CO—) ou méthylène ($—CH_2—$); et $R^{10}$ est un groupe hydroxy, amino, aryloxy, arylthio, alkoxy, cycloalkoxy, alkylamino, dialkylamino, amino cyclique, hydroxyalkoxy, acyloxyalkoxy, aminoalkoxy, alkylaminoalkoxy, dialkylaminoalkoxy, aminoalkoxy cyclique ou arylalkoxy dont chaque radical alkyle ou alkoxy a jusqu'à 5 atomes de carbone et le groupe amino cyclique a jusqu'à 6 atomes de carbone; ou bien $R^{10}$ et $R^6$ s'associent de façon telle que $R^{10}$ représente de l'oxygène (—O—) lié à l'atome terminal de carbone de $R^6$ lorsqu'il s'agit d'un groupe alkyle; $R^{11}$ est l'hydrogène ou un groupe alkyle ayant jusqu'à 3 atomes de carbone; X est un groupe carbonyle (—CO—), hydroxyméthylène (—CHOH—), thiocarbonyle (—CS—) ou oximinométhylène ($—C=N—OR^{20}$, où $R^{20}$ est l'hydrogène ou un radical alkyle ayant jusqu'à 5 atomes de carbone); et Y est un groupe carbonyle, thiocarbonyle, sulfonyle ($—SO_2$) ou amino (—NHCO—); ou un sel de ce dérivé dans les cas appropriés.

2. Dérivé amidique suivant la revendication 1, dans lequel X, Y et Q sont tous des groupes carbonyle et $R^2$ et $R^{11}$ sont tous de l'hydrogène, ou un sel de ce dérivé.

3. Dérivé amidique de formule:

$$R^1CONH—CHR^3—COCH_2—CHR^4—CONR^5—CHR^6—COR^{10}$$

dans laquelle $R^1$ est un groupe méthyle, benzyle, β-phényl-éthyle, benzyloxy, phénoxyméthyle, phénylthiométhyle, phényle, tolyle, méthoxyphényle, méthylthiophényle, monochlorophényle, dichlorophényle, fluorophényle, iodophényle, nitrophényle, cyanophényle, trifluorométhylphényle, acétamidophényle, acétylphényle, méthanesulfonylphényle, biphényle, naphtyle, benzamidométhyle, cyclopentanecarboxamidométhyle ou β-benzoyléthyle; $R^3$ est un groupe méthyle substitué par un noyau hétérocyclique pentagonal ou hexagonal entièrement insaturé contenant 1 ou 2 hétéro-atomes choisis entre des atomes d'oxygène, d'azote et de soufre, ce noyau pouvant éventuellement contenir un

substituant méthyle ou pouvant être condensé avec un noyau benzénique, sous réserve que $R^3$ ne soit pas un groupe indolylméthyle; $R^4$ est l'hydrogène ou un groupe méthyle; $R^5$ est l'hydrogène ou un groupe méthyle et $R^6$ est l'hydrogène, un groupe benzyle ou un groupe indole-3-yl-méthyle, ou bien $R^5$ et $R^6$ forment conjointement un groupe triméthylène, tétraméthylène, pentaméthylène, 2-thiatriméthylène, 3-thiatétraméthylène, 3-oxatétraméthylène, 3-méthyl-3-azatétraméthylène; et $R^{10}$ est un groupe hydroxy, amino, alkoxy ayant jusqu'à 5 atomes de carbone, β-diméthylaminoéthoxy, β-morpholinoéthoxy ou benzyloxy, ou un sel de ce dérivé.

4. Dérivé amidique suivant la revendication 3, dans lequel $R^1$ est un groupe phényle, $R^3$ est un groupe méthyle substitué par un radical 3-thiényle, 5-méthyl-2-furyle, 2-pyridyle, 3-pyridyle, 4-thiazolyle, 4-imidazolyle, 3-isoxazolyle ou 2-benzimidazolyle, $R^4$ est l'hydrogène ou un groupe méthyle, $R^5$ et $R^6$ forment conjointement un groupe triméthylène et $R^{10}$ est un groupe hydroxy, alkoxy ayant jusqu'à 5 atomes de carbone ou benzyloxy, ou un sel de ce dérivé.

5. Dérivé amidique de formule:

$$R^1CONH—CHR^3—COCH_2—CHR^4—CONR^5—CHR^6—COR^{10}$$

dans laquelle $R^1$ est un noyau hétérocyclique pentagonal ou hexagonal entièrement insaturé contenant 1 ou 2 hétéroatomes choisis entre des atomes d'oxygène, d'azote et de soufre, ce noyau pouvant éventuellement contenir un substituant méthyle ou pouvant être condensé avec un noyau benzénique; $R^3$ est un groupe benzyle qui n'est pas substitué ou qui porte un substituant méthyle, tertio-butyle, méthoxy, fluoro, chloro, bromo, nitro, cyano, trifluorométhyle, amino, acétamido, méthanesulfamido, trifluorométhanesulfamido ou phényle; ou bien $R^3$ est un groupe 3-phényle ou 3-phénylеprop-2-ényle; $R^4$ est l'hydrogène ou un groupe méthyle; $R^5$ est l'hydrogène ou un groupe méthyle et $R^6$ est l'hydrogène, un groupe benzyle ou un groupe indole-3-ylméthyle, ou bien $R^5$ et $R^6$ forment conjointement un groupe triméthylène, tétraméthylène, pentaméthylène, 2-thiatriméthylène, 3-thiatétraméthylène, 3-oxatétraméthylène ou 3-méthyl-3-azatétraméthylène; et $R^{10}$ est un groupe hydroxy, amino, alkoxy ayant jusqu'à 5 atomes de carbone, β-diméthylaminoéthoxy, β-morpholinoéthoxy ou benzyloxy, ou un sel de ce dérivé.

6. Dérivé amidique suivant la revendication 5, dans lequel $R^1$ est un groupe 2-furyle, 2-thiényle ou 2-pyridyle, $R^3$ est un groupe benzyle, $R^4$ est l'hydrogène ou un groupe méthyle, $R^5$ et $R^6$ forment conjointement un groupe triméthylène et $R^{10}$ est un groupe hydroxy ou alkoxy ayant jusqu'à 5 atomes de carbone, ou un sel de ce dérivé.

7. La *N*-[2-méthyle-4-oxo-6-phényl-5-(2-thénoyl-amino)hexanoyl]-L-proline; l'ester isobutylique de la *N*-[5-(2-furoylamino)-4-oxo-6-phénylhexanoyl]-L-proline; la *N*-[5-benzamido-4-oxo-6-(pyrid-3-yl)hexanoyl]-L-proline ou la *N*-[5-benzamido-6-(imidazole-4-yl)-4-oxohexanoyl]-L-proline.

8. Sel d'un dérivé amidique suivant l'une quelconque des revendications 1 à 7, dans lequel $R^{10}$ est un groupe hydroxy qui est un sel de métal alcalin ou de métal alcalino-terreux, ou un sel d'ammonium ou de dicyclohexylamine.

9. Procédé de production d'un dérivé amidique ou d'un sel de ce dérivé suivant l'une quelconque des revendications 1 à 8, qui comprend la réaction d'un acide de formule:

$$R^1—Y—NR^2—CHR^3—X—CHR^{11}—CHR^4—COOH$$

dans laquelle $R^1$, $R^2$, $R^3$, $R^4$, $R^{11}$, X et Y ont les définitions données dans l'une quelconque des revendications 1 à 5, ou d'un dérivé réactif de cet acide, avec une amine de formule:

$$HNR^5—CH^6R^{16}—Q—R^{12}$$

dans laquelle $R^5$, $R^6$, $R^{16}$ et Q ont les définitions données dans l'une quelconque des revendications 1 à 5 et $R^{12}$ a l'une quelconque des définitions données dans les revendications 1 à 5 pour $R^{10}$, excepté celles qui comportent un groupe hydroxy ou amino libre; après quoi un dérivé amidique dans lequel $R^2$ est un groupe alkyle, arylalkyle ou aryle (et $R^{10}$ a la valeur de $R^{12}$) peut être obtenu par réaction du dérivé amidique correspondant dans lequel $R^2$ est l'hydrogène (et $R^{10}$ a la valeur de $R^{12}$) avec un composé de formule $R^2Z$, dans laquelle $R^2$ a la définition donnée ci-dessus et Z est un halogène, un groupe alcanesulfonyle ou un groupe arènesulfonyle déplaçables; ou bien un dérivé amidique dans lequel Q est un groupe carbonyle et $R^{10}$ est un groupe hydroxy peut ensuite être obtenu par hydrolyse du dérivé amidique correspondant dans lequel $R^{10}$ est un groupe alkoxy ou bien, lorsque $R^{10}$ est un groupe tertio-butoxy, par le clivage, catalysé par un acide, dudit compose; ou bien un dérivé amidique dans lequel X est un groupe hydroxyméthylène peut être obtenu ensuite par réduction du dérivé amidique correspondant dans lequel X est un groupe carbonyle; ou bien un dérivé amidique dans lequel X est un groupe oximinométhylène peut ensuite être obtenu par réaction du dérivé amidique correspondant dans lequel X est un groupe carbonyle, avec l'hydroxylamine ou un dérivé d'hydroxylamine substitué sur *O*.

10. Composition pharmaceutique comprenant comme ingrédient actif au moins un dérivé amidique ou un sel de ce dérivé suivant l'une quelconque des revendications 1 à 8, en association avec un diluant ou support pharmaceutiquement acceptable.